# EUROPEAN PATENT APPLICATION

(11) **EP 1 293 195 A1**
(43) Date of publication of application: **19.03.2003**
(21) Application number: 02256157.5
(22) Date of filing: 05.09.2002
(51) Int. Cl.: A61K 9/20, A61K 9/50, A61K 45/06, A61K 31/485

(54) **Oral pharmaceutical dosage forms with reduced potential for drug abuse, comprising respiratory irritants or bitter substances**

(30) Priority: 17.09.2001 US 322624 P; 02.11.2001 US 16336
(71) Applicant: ROHM AND HAAS COMPANY, Philadelphia, Pennsylvania 19106-2399 (US)
(72) Inventor: Hugues, Lyn, Harleysville, Pennsylvania 19438 (US); Bellamy, Simon Andrew, Redhill, Surrey RH1 6DD (GB)
(74) Representative: Kent, Venetia Katherine

(57) **Abstract**

Solid oral dosage forms of controlled substances containing aversive agents are useful in reducing abuse by chewing or inhaling.

## Description

### BACKGROUND

Abuse of controlled substances is a serious and growing problem throughout the world. For example, the abuse of an extended release form of oxycodone has been the recent subject of many articles such as 'Playing With Pain Killers' and 'How One Town Got Hooked'. See, Newsweek, April 9, 2001, pages 45-51. Further, The New York Times profiled the problem of oxycodone abuse by inhalation of the crushed pill. See, The New York Times, July 29, 2001. It is estimated that in America four million people over the age of 12 used prescription painkillers and stimulants for non-medical reasons in the space of just one month, approximately half of them saying they'd done it for the first time. Emergency room visits related to such abuse approximately doubled between 1992 and 1999.

There are three main routes that drug abusers use for administering the drug substances: parenteral, oral, and inhalation. The parenteral route is commonly called 'mainlining' and requires the drug substance to be in solution such that it can be injected intravenously with a syringe. For solid dosage form drugs this requires some type of extraction and concentration procedure to render the drug substance suitable for injection. Inhalation of a solid drug substance through the nose is commonly called 'snorting'. For solid dosage form drugs this requires only that the dosage form be crushed into a powder, or emptied from a capsule. Breathing in vapors is frequently known as 'huffing'. Both snorting and huffing result in the rapid absorption of the drug substance through the mucosa of the respiratory system.

The potential for abuse is increased by the use of extended release formulations because they typically contain more than the immediate release single dose of active ingredient. Circumventing the extended release mechanism delivers the full dose, which is intended to be delivered over a longer time period, immediately. For example, crushing an extended release oxycodone tablet separates a gelling matrix from the oxycodone active ingredient, such that when inhaled through the nose the gelling matrix cannot exert the extended release effect. Similarly it is sometimes possible to circumvent the extended release effect by chewing the dosage form.

The use of coatings to extend the release of drug substances is very well known in the art (Remington's Pharmaceutical Sciences, 16^{th} Edition, Chapter 90). Such dosage forms are also subject to said modes of abuse because the coating can be damaged by crushing or chewing.

WO0108661 describes an extended release dosage form of opioids that uses an ion exchange resin. This dosage form is also subject to said modes of abuse because the ion exchange resin and the active ingredient can be separated by crushing.

Various methods have been used to reduce the potential for abuse of controlled substances. These methods have focussed on the parenteral and oral routes of administration.

US3,773,955, US3,966,940, and US4,457,933 describe oral dosage forms containing a combination of opioid agonists and antagonists, in which the effect of the antagonist when administered according to the correct procedure does not affect the therapeutic pain management value of the agonist. However, when the agonist and antagonist are extracted for parenteral administration by an addict the effect of the agonist desired by the addict is decreased. This approach was further adopted in WO9004965 where it was incorporated into a transdermal delivery device, and in US 6,228,863 where it was developed into a dosage form from which the agonist could not be separated from the antagonist except by using a sophisticated multi-step procedure.

In US 3,980,766 multiple methods for reducing abuse potential are described. One method is to include a thickening agent such that the concentrated extract containing the drug can not be injected with a syringe. Another is the incorporation of agents that cause the precipitation of the drug during isolation, thus rendering it unsuitable for injection. The addition of a thickener has also been used in US 4,070,494, WO9107950, and WO9520947.

In WO0033835 additives are included in the dosage forms such that when added to drinks create a visible change in the drink. This invention reduces the potential for abuse by oral administration of the substance by one person to another without their knowledge.

The use of bitter and sour agents to minimize the risk of ingestion of poisonous compounds is well known in the art. For example, see US 3,268,577, GB 2358585, JP 2000026260, and Chemistry and Industry (London), volume 22, 721-723, 1998). In all such cases the purpose of the bitter or sour agent is to prevent ingestion.

However, none of the cited references solve the problem of potential abuse of therapeutic compounds via inhalation or chewing. Now, Applicants have surprisingly discovered a dosage form useful in reducing the potential for drug abuse via inhalation or chewing.

The term "high," as used herein means the non-therapeutic effect desired by drug addicts and recreational drug users

The term "respiratory mucosal membrane" as used herein means the mucous membrane lining the nasal and pharyngeal cavities, the bronchial tubes, and the lungs. Typically, snorting into the nasal cavity is the common, preferred route of abuse for a solid oral dosage form which has been crushed by one intending to inhale said crushed dosage form to obtain the high.

The term "respiratory irritant' as use herein means substances that cause irritation when administered to the respiratory mucosal membrane. Said irritation can include, but is not limited to, coughing, dyspnea, rhinitis, nasal congestion, eye irritation, lachrymation, and sneezing.

When describing dosage forms the term "immediate release" as used herein means a dosage form from which the active ingredient is dissolved as quickly as possible after administration. In the pharmaceutical arts said immediate release dosage forms are frequently referred to as "conventional" dosage forms.

When describing dosage forms the term "modified release" as used herein means a dosage form whose drug-release characteristics of time course and/or location are chosen to accomplish therapeutic or convenience objectives not offered by conventional dosage forms. Said modified release dosage forms include dosage forms commonly known in the art as, delayed, sustained, extended, targeted, prolonged, pulsatile, zero-order, constant rate, and controlled.

The term "aversive response" as used herein means a response in a person, resulting from administration of a dosage form containing a controlled substance, via any of the known routes of administration, sufficiently unpleasant that said person decides not to administer said dosage form by the same route of administration again

The term "aversive agent" as used herein means any substance that is included in a dosage form that creates an aversive response.

The term "nociceptive" as used herein means a response characterized by pain. For example the term 'nociceptive efficacy' when applied to an irritant refers to the quantification of the ability of said irritant to cause pain.

### SUMMARY OF THE INVENTION

The present invention relates to an oral pharmaceutical dosage form not susceptible to abuse by respiratory mucosal membrane administration comprising one or more aversive agents.

The present invention further relates to an oral pharmaceutical dosage form not susceptible to abuse by chewing comprising one or more aversive agents.

### DETAILED DESCRIPTION OF THE INVENTION

The present invention relates to an oral pharmaceutical dosage form not susceptible to abuse by respiratory mucosal membrane administration comprising one or more aversive agents.

The present invention further relates to an oral pharmaceutical dosage form not susceptible to abuse by chewing comprising one or more aversive agents.

In the practice of one embodiment of the invention, a respiratory irritant such as powdered chili peppers, or concentrated extracts of such products that contain capsaicin or capsaicin-like components, is incorporated into the solid oral dosage form of the controlled substance. When the oral dosage form is used as prescribed, i.e. swallowed whole, said irritant causes no aversive response. However, if the oral dosage form is rendered into a powder and inhaled, said irritant creates intense discomfort in the user, including coughing, dyspnea, rhinitis, nasal congestion, eye irritation, lachrymation, and sneezing. This intense discomfort has the effect of deterring people from using said inhalation route as a means of administration, i.e. it elicits an aversive response.

In the practice of another embodiment of the invention, a bitter tasting agent such as denatonium benzoate (Bitr
ex®) or a sour tasting agent such as citric acid , is incorporated into the solid oral dosage form of the controlled substance. When the oral dosage form is used as prescribed, i.e. swallowed whole, said bitter or sour substance causes no aversive response. However, if the oral dosage form is chewed, said bitter or sour substance creates an intensely unpleasant taste. This unpleasant taste has the effect of deterring people from chewing the dosage form, i.e. it elicits an aversive response.

The Controlled Substances Act of 1970 regulates the manufacturing, distribution, and dispensing of drugs that have abuse potential. The Drug Enforcement Agency (DEA) within the US Department of Justice is the chief agency responsible for enforcing said act. Drugs under the jurisdiction of said Act are divided into five schedules (I thru V) based on their medical utility, potential for abuse, and physical and psychological dependence. Schedule I substances have high abuse potential and no accepted medical use. Schedule II also have high abuse potential, but also have medical utility. Schedules III, IV, and V have progressively lower abuse potential.

Because the DEA rates abuse potential based on specific dosage forms it is not uncommon for a drug to be rated in multiple schedules. For example codeine appears as Schedule II, Schedule III, and Schedule IV, depending on the specific dosage form and dosage amount. To avoid duplication in the list of controlled substance below, multiple occurrences have been removed and any controlled substance that had multiple occurrences is placed in the highest abuse potential category for which is has been scheduled. For example, codeine has been included as Schedule II, but not Schedule III or Schedule IV. This is not intended to limit the scope of the invention. The utility of the Applicant's invention lies in the fact that any controlled substance, regardless of what schedule it appears on, is suitable for formulating into the Applicant's dosage form.

Controlled substances useful in the practice of the invention are those categorized by the DEA as Schedule II, III, IV, and V controlled substances.

Schedule II substances include, but are not limited to, 1-1-Phenylcyclohexylamine, 1-Piperidinocyclohexanecarbonitrile, Alfentanil, Alphaprodine, Amobarbital, Amphetamine, Anileridine, Benzoylecgonine, Bezitramide, Carfentanil, Coca Leaves, Cocaine, Codeine, Dextropropoxyphene, Dihydrocodeine, Diphenoxylate, Diprenorphine, Ecgonine, Ethylmorphine, Etorphine HCl, Fentanyl, Glutethimide, Hydrocodone, Hydromorphone, Isomethadone, Levo-alphacetylmethadol, Levomethorphan, Levorphanol, Meperidine, Meperidine intermediate-A, Meperidine intermediate-B, Meperidine intermediate-C, Metazocine, Methadone, Methadone intermediate, Methamphetamine, Methylphenidate, Metopon, Moramide-intermediate, Morphine, Nabilone, Opium extracts, Opium fluid extract, Opium poppy, Opium tincture, Opium, granulated, Opium, powdered, Opium, raw, Oxycodone, Oxymorphone, Pentobarbital, Phenazocine, Phencyclidine, Phenmetrazine, Phenylacetone, Piminodine, Poppy Straw, Poppy Straw Concentrate, Racemethorphan, Racemorphan, Remifentanil, Secobarbital, Sufentanil, Thebaine

Schedule III substances include, but are not limited to, Amobarbital, Anabolic steroids, Aprobarbital, Barbituric acid derivative, Benzphetamine, Boldenone, Butabarbital, Butalbital, Chlorhexadol, Chlorotestosterone, Chlorphentermine, Clortermine, Clostebol, Codeine, Dehydrochlormethyltestosterone, Dihydrocodeine, Dihydrotestosterone, Dronabinol, Drostanolone, Ethylestrenol, Ethylmorphine, Fluoxymesterone, Formebolone, Hydrocodone, Ketamine, Lysergic acid, Lysergic acid amide, Mesterolone, Methandienone, Methandranone, Methandriol, Methandrostenolone, Methenolone, Methyltestosterone, Methyprylon, Mibolerone, Morphine, Nalorphine, Nandrolone, Norethandrolone, Oxandrolone, Oxymesterone, Oxymetholone, Pentobarbital, Phendimetrazine, Secobarbital, Stanolone, Stanozolol, Sulfondiethylmethane, Sulfonethylmethane, Sulfonmethane, Talbutal, Testolactone, Testosterone, Thiamylal, Thiopental, Tiletamine, Trenbolone, Vinbarbital.

Schedule IV substances include, but are not limited to, Alprazolam, Barbital, Bromazepam, Butorphanol, Camazepam, Cathine, Chloral betaine, Chloral hydrate, Chlordiazepoxide, Clobazam, Clonazepam, Clorazepate, Clotiazepam, Cloxazolam, Cocaine, Delorazepam, Dexfenfluramine, Dextropropoxyphene, Diazepam, Diethylpropion, Difenoxin, Estazolam, Ethchlorvynol, Ethinamate, Ethyl loflazepate, Fencamfamin, Fenfluramine, Fenproporex, Fludiazepam, Flunitrazepam, Flurazepam, Halazepam, Haloxazolam, Ketazolam, Loprazolam, Lorazepam, Lormetazepam, Mazindol, Mebutamate, Medazepam, Mefenorex, Meprobamate, Methohexital, Methylphenobarbital, Midazolam, Modafinil, Nimetazepam, Nitrazepam, Nordiazepam, Oxazepam, Oxazolam, Paraldehyde, Pemoline, Pentazocine, Petrichloral, Phenobarbital, Phentermine, Pinazepam, Pipradrol, Prazepam, Quazepam, Sibutramine, Temazepam, Tetrazepam, Triazolam, Zaleplon, Zolpidem

Schedule V substances include, but are not limited to Buprenorphine, Difenoxin, Dihydrocodeine, Diphenoxylate, Pyrovalerone.

Preferred controlled substances useful in the practice of the invention are those categorized by the DEA as Schedule II, III, and IV controlled substances.

More preferred controlled substance useful in the practice of the invention are those categorized by the DEA as Schedule II and III controlled substances.

Most preferred controlled substance useful in the practice of the invention are those categorized by the DEA as Schedule II controlled substances. The most preferred Schedule II substance is oxycodone.

Aversive agents useful in the practice of this invention include, but are not limited to, respiratory irritants, bitter substances, and sour substances.

Aversive agents useful in the practice of this invention are solids. Said solid can be said agent in pure form or a solid containing said agent.

Aversive agents useful in the practice of this invention are of natural or synthetic origin.

An important aspect of this invention is the use of capsaicinoids as an aversive agent which acts as a respiratory irritant to create an aversive response. Capsaicinoids are alkaloid substances which occur naturally in the fruit of various chile pepper plants. The principal capsaicinoids found in most pepper plants are capsaicin, dihydrocapsaicin, capsico, and capsacutin. The principal capsaicinoid is capsaicin. There can be multiple capsaicinoids in one pepper and different peppers have different concentrations of capsaicinoids. The production of capsaicinoids is a form of chemical defense against being eaten and thus acts naturally as an animal repellant. See, Smith, R. L., Ecology and Field Biology, p. 562 (3d Ed. 1980). Capsaicinoids are the chemicals responsible for the "hot" sensation associated with peppers. The hotness of the various capsicums is directly attributable to their capsaicinoid content. Capsaicinoids generate a spicy flavor in the mouth but are irritants when applied to mucous membranes.

Capsicum is the formal term used to refer to the dried ripe fruit of the various species of chili peppers.

Therapeutically, capsaicin is listed as a counterirritant (Merck Index, 9th Ed., p. 224). Capsicum has Generally Regarded as Safe (GRAS) status in the USA. Capsaicin, capsicum, and capsicum oleoresin have monographs in the US Pharmacopeia 24.

Respiratory irritants useful in the practice of this invention include, but are not limited to, pure compounds and mixtures of capsaicin, capsico, capsacutin, dihydrocapsaicin, nordihydrocapsaicin, homocapsaicin, homodihydrocapsaicin, capsaicinoids, gingerol, chemical mace, piperine, isochavicine, isopiperine, piperidine, chavicine, piperettine, zingerone, shogaol, valleral, isovallerals, vanyllylamide, nonoyl vanyllamide, vanyllylamide derivatives, synthetic derivatives of capsaicinoids, and extracts, capsicums, and powders of, Capsicum frutescens varieties, Capsicum anuum varieties, Capsicum chinense varieties, Capsicum baccatum varieties, Capsicum pubescens varieties, Capsicum species, Piper migrum varieties, Piper longum varieties, Piper retrofractum varieties, Piper officinarum varieties, Piperaceae species, Brassica juncea varieties, Brassica. nigra varieties, Sinapis alba varieties, Sinapis arvensis varieties, Zingiber officinale varieties, and Lactarius vellereus varieties and mixtures thereof.

Preferred respiratory irritants useful in the practice of the invention are pure compounds and mixtures of capsaicin, capsico, capsacutin, dihydrocapsaicin, nordihydrocapsaicin,homocapsaicin, homodihydrocapsaicin, capsaicinoids, gingerol, chemical mace, piperine, isochavicine, isopiperine, piperidine, chavicine, piperettine, zingerone, shogaol, valleral, isovallerals, vanyllylamide, nonoyl vanyllamide, vanyllylamide derivatives, synthetic derivatives of capsaicinoids, and extracts, capsicums, and powders of, Capsicum frutescens varieties, Capsicum anuum varieties, Capsicum chinense varieties, Piper migrum varieties, Piper longum varieties, Piper retrofractum varieties, Piper officinarum varieties, Brassica juncea varieties, Brassica. nigra varieties, Sinapis alba varieties, Sinapis arvensis varieties, and Zingiber officinale varieties and mixtures thereof.

More preferred respiratory irritants useful in the practice of the invention are pure compounds and mixtures of capsaicin, capsico, capsacutin dihydrocapsaicin, nordihydrocapsaicin,homocapsaicin, homodihydrocapsaicin, capsaicinoids, gingerol, piperine, isopiperine, piperidine, piperettine, zingerone, shogaol, valleral, isovallerals, vanyllylamide, vanyllylamide derivatives, and extracts, capsicums, and powders of, Capsicum frutescens varieties, Capsicum anuum varieties, Capsicum chinense varieties, Piper migrum varieties, Piper longum varieties, Piper retrofractum varieties, Piper officinarum varieties, Brassica juncea varieties, Brassica. nigra varieties, Sinapis alba varieties, Sinapis arvensis varieties, and Zingiber officinale varieties and mixtures thereof.

Most preferred respiratory irritants useful in the practice of the invention are pure compounds and mixtures of capsaicin, capsacutin dihydrocapsaicin, nordihydrocapsaicin, homocapsaicin, homodihydrocapsaicin, capsaicinoids, gingerol, piperine, isopiperine, zingerone, shogaol, and vanyllylamide derivatives and mixtures thereof.

The use of capsaicin with cocaine is contra-indicated.

The amount of respiratory irritant useful in the practice of this invention is that which is sufficient to elicit an aversive response in the user when said irritant is inhaled through the respiratory mucosa but that which is not sufficient to elicit an aversive response or an adverse medical response in the user when said irritant is swallowed as a solid oral dosage form in the manner prescribed.

The nociceptive efficacy of the respiratory irritants varies greatly depending both on chemical structure of the active ingredient of said irritant, and the amount of active ingredient in said irritant. The following amounts of respiratory irritants are provided as examples. Effective amounts of other respiratory irritants can be determined using techniques well known to those skilled in the art.

The preferred amount of the respiratory irritants capsaicin and dihydrocapsaicin is a combined total of 0.002-100mg per dose.

The preferred amount of the respiratory irritant zingerone is 0.04-200mg per dose.

The preferred amount of the respiratory irritant shogaol is 0.04-200mg per dose

The preferred amount of the respiratory irritant piperine is 0.04-200mg per dose

The preferred amount of the respiratory irritants capsicums of Capsicum annum, Capsicum frutescens, and Capsicum chinense is 0.1 - 450mg per dose.

The more preferred amount of the respiratory irritants capsaicin and dihydrocapsiacin is a combined total of 0.004-25mg per dose.

The more preferred amount of the respiratory irritant zingerone 0.2-150mg per dose.

The more preferred amount of the respiratory irritant shogaol 0.2-150mg per dose

The more preferred amount of the respiratory irritant piperine is 0.2-150mg per dose

The more preferred amount of the respiratory irritants capsicums of Capsicum annum, Capsicum frutescens, and Capsicum chinense is 0.4-350mg per dose

The most preferred amount of the respiratory irritants capsaicin and dihydrocapsiacin is a combined total of 0.02-15mg per dose.

The most preferred amount of the respiratory irritant zingerone 0.2-100mg per dose.

The most preferred amount of the respiratory irritant shogaol 0.2-100mg per dose

The most preferred amount of the respiratory irritant piperine is 0.2-100mg per dose

The most preferred amount of the respiratory irritants capsicums of Capsicum annum, Capsicum frutescens, and Capsicum chinense is 0.6-250mg per dose

Bitter agents also create an aversive response. The use of bitter agents is particularly useful in preventing abuse of controlled substances by chewing the oral dosage form. Bitter agents useful in the practice of this invention include, but are not limited to, agaricic acid, benzyl acetate, brucine, brucine sulfate, caffeine, capsaicin, catechin, dadzein, denatonium benzoate (Bitrex®) and other denatonium salts, denatonium capsaicinate, denatonium chloride, denatonium saccharide, diethyl phthalate, epicatechin, genistein, gentian violet, gerianol, hydroxytyrosol, kashin, limonin, linalool, linalool acetate, methyl anthranilate, naringin, nobiletin, oleuropin, phenylethyl alcohol, polyphenols, quassin, quebracho, quercitin, quinine, quinine sulfate, quinine hydrochloride, sinensetin, sucrose benzoate, sucrose octaacetate, and tangeretin and mixtures thereof.

Preferred bitter agents useful in the practice of this invention are, denatonium benzoate (Bitrex®) and other denatonium salts, denatonium capsaicinate, denatonium chloride, denatonium saccharide, limonin, linalool, linalool acetate, naringin, quassin, quercitin, sucrose benzoate, and sucrose octaacetate and mixtures thereof.

Most preferred bitter agents useful in the practice of this invention are denatonium benzoate (Bitrex®), denatonium capsaicinate, denatonium saccharide, and sucrose octaacetate and mixtures thereof.

Further, sour agents also create an aversive response. The use of sour agents is particularly useful in preventing abuse of controlled substances by chewing the oral dosage form. Sour agents useful in the practice of this invention include, but are not limited to, acidic organic compounds that contain one or more acidic protons per molecule and mixtures thereof.

Preferred sour agent useful in the practice of the invention are acidic organic compounds that contain two or more acidic protons per molecule and mixtures thereof.

Most preferred sour agents useful in the practice of the invention are citric acid and tartaric acid and mixtures thereof.

The controlled substance and aversive agent are incorporated into the dosage form using any of the methods known in the art for preparation of solid oral dosage forms. See, Remington's Pharmaceutical Sciences, 16^{th} Edition.

Further, different combinations of aversive agents can be combined in the same dosage form. For example, if it is desired to reduce the potential for abuse via both inhalation and chewing, it may be desirable to combine both a respiratory irritant and a bitter agent in the same formulation. Further, combination of avervise agents can sometime have a synergistic effect, such that the combination has a greater effect than the sum of the individuals taken separately. Still further, people have different responses to taste, such that a mixture of bitter agents may be needed to be effective in a larger fraction of the population.

In addition to the controlled substance and aversive agent, excipients are used in the manufacture of the oral dosage forms of the present invention. Excipients useful in the practice if this invention include but are not limited to preservatives, viscosity agents, fillers, lubricants, glidants, disintegrants, binders, and encapsulants.

Preferred preservatives include, but are not limited to, phenol, alkyl esters of parahydroxybenzoic acid, o-phenylphenol benzoic acid and the salts thereof, boric acid and the salts thereof, sorbic acid and the salts thereof, chlorobutanol, benzyl alcohol, thimerosal, phenylmercuric acetate and nitrate, nitromersol, benzalkonium chloride, cetylpyridinium chloride, methyl paraben, and propyl paraben. Particularly preferred are the salts of benzoic acid, cetylpyridinium chloride, methyl paraben and propyl paraben. The compositions of the present invention generally include from 0-2% preservatives.

Preferred viscosity agents include, but are not limited to, methylcellulose, sodium carboxymethylcellulose, hydroxypropyl-methylcellulose, hydroxypropylcellulose, sodium alginate, carbomer, povidone, acacia, guar gum, xanthan gum and tragacanth. Particularly preferred are methylcellulose, carbomer, xanthan gum, guar gum, povidone, sodium carboxymethylcellulose, and magnesium aluminum silicate. Compositions of the present invention include 0-25% viscosity agents.

Preferred fillers include, but are not limited to, lactose, mannitol, sorbitol, tribasic calcium phosphate, dibasic calcium phosphate, compressible sugar, starch, calcium sulfate, dextro and microcrystalline cellulose. The compositions of the present invention contain from 0-75% fillers.

Preferred lubricants include, but are not limited to, magnesium stearate, stearic acid, and talc. The pharmaceutical compositions of the present invention include 0-2% lubricants.

Preferred glidants include, but are not limited to, talc and colloidal silica. The compositions of the present invention include from 0-5% glidants.

Preferred disintegrants include, but are not limited to, starch, sodium starch glycolate, crospovidone, croscarmelose sodium, polacrilin potassium, and microcrystalline cellulose. The pharmaceutical compositions of the present invention include from 0-30% disintegrants.

Preferred binders include, but are not limited to, acacia, tragacanth, hydroxypropylcellulose, pregelatinized starch, gelatin, povidone, hydroxypropylcellulose, hydroxypropyl-methylcellulose, methylcellulose, sugar solutions, such as sucrose and sorbitol, and ethylcellulose. The compositions of the present invention include 0.1-10% binders.

Encapsulants useful in the practice of the present invention include, but are not limited to permable coatings, impermeable coatings, and matrices.

Permeable coatings useful in this invention are well know to one skilled in the art and include Eudragit® RL, and Eudragit® RS (Rohm-Pharma Darmstadt, Germany)

Non-permeable coatings useful in this invention are well known to one skilled in the art and include Aquacoat CPD (FMC Corporation, Philadelphia, PA, USA), Eudragit® E100, Eudragit® L100, Eudragit® S100 (Rohm-Pharma Darmstadt, Germany), Kollicoat® MA 30 DP (BASF Aktiengesellschaft, Ludwigshafen, Germany), Opadry light pink.

Plastizers for use with coatings useful in the practice of the invention include but are not limited to, triethyl citrate, 1,2-propylene glycol, polyethylene glycols, and tracetin.

Matrices for encapsulation useful in the practice of the invention include, but are not limited to, anion exchange resins, cation exchange resins, polymeric adsorbents, carbonaceous adsorbents, cellulosic polymers, and acrylic polymers.

Dosage forms of the present invention are immediate release or modified release.

The following non limiting examples illustrate the present invention:

### EXAMPLE 1 30mg Extended release oxycodone tablets using capsaicin

| Ingredient | mg/tabl et |
|---|---|
| Oxycodone HCl | 30 |
| Lactose | 200 |
| Eudragit® RS PM | 45 |
| Purified water | as needed |
| Stearyl alcohol | 75 |
| Talc | 7.5 |
| Magnesium stearate | 3.75 |
| Capsaicin | 13.75 |

The required quantities of oxycodone hydrochloride, a Schedule II controlled substance, spray-dried lactose, capsaicin, and Eudragit® RS PM are placed into an appropriately-sized mixer, and mixed for approximately 5 minutes. While the powders are mixing, the mixture is granulated with enough water to produce a moist granular mass. The granules are then dried in a fluid bed dryer at 60° C., and then passed through an 8-mesh screen. Thereafter, the granules are redried and pushed through a 12-mesh screen. The required quantity of stearyl alcohol is melted at approximately 60-70° C., and while the granules are mixing, the melted stearyl alcohol is added. The warm granules are returned to the mixer.

The coated granules are removed from the mixer and allowed to cool. The granules are then passed through a 12-mesh screen. The granulate is then lubricated by mixing the required quantity of talc and magnesium stearate in a suitable blender. Tablets are compressed to 375 mg in weight on a suitable tableting machine.

### EXAMPLE 2 30mg Extended release morphine sulfate tablets using capsaicin

| Ingredient | mg/tabl et |
|---|---|
| Morphine sulfate | 30 |
| Lactose | 79.5 |
| Eudragit® RL | 12 |
| Stearyl aclohol | 24 |
| Talc | 3 |
| Magnesium stearate | 1.5 |
| Capsaicin | 10 |

These tablets are prepared according to the following method:

Morphine sulfate, a Schedule II controlled substance, capsaicin, and lactose are intimately mixed in a suitable mixer. A granulation is then prepared by incorporating the granulating fluid into the mixing powders using Eudragit® RL. The granulate is then dried and passed through a 12 mesh screen. The stearyl alcohol is melted and incorporated into the warm granules using a suitable mixer. After cooling, the granules are passed through a 12 mesh screen. The granules are then lubricated by mixing in the talc and stearyl alcohol. Tablets are then compressed on a suitable tabletting machine using round biconvex tooling 10/32" in diameter.

### EXAMPLE 3 8mg extended release Hydromorphone capsules using shogaol

| Ingredient | mg/capsu le |
|---|---|
| Loading | |
| Hydromorphone HCl | 8 |
| Opadry light pink (Y-5-1442) | 4 |
| Purified water | as needed |
| 18/20 mesh sugar spheres | 148 |
| | |

| Overcoating | |
|---|---|
| Opadry light pink (Y-5-1442) | 8.4 |
| Purified water | as needed |
| | |

| Retardant coating | |
|---|---|
| Eudragit® RS 30D | 7.6 |
| Eudragit® RL 30D | 0.8 |
| Triethyl citrate | 1.68 |
| Talc | 3.36 |
| Purified water | as needed |
| | |

| Second overcoat | |
|---|---|
| Opadry light pink (Y-5-1442) | 9.6 |
| Purified water | ass needed |
| | |

| Encapsulation | |
|---|---|
| Size #2 clear hard gelatin capsules | |
| Shogaol | 75 |

Hydromorphone beads are prepared by dissolving hydromorphone HCl, a Schedule II controlled substance, in water, adding Opadry Y-5-1442 and mixing for about 1 hour to obtain a 20% w/w suspension. This suspension is then sprayed onto Nu-Pareil® 18/20 mesh beads using a Wurster insert.

### First Overcoat

The loaded hydromorphone beads are then overcoated with a 5% w/w gain of Opadry Light Pink using a Wurster insert. This overcoat is applied as a protective coating.

### Retardant Coat

After the first overcoat, the hydromorphone beads are then coated with a 5% weight gain of a retardant coating mixture of Eudragit® RS 30D and Eudragit® RL 30D at a ratio of 90:10, RS to RL. The addition of Triethyl Citrate and Talc is also included in the Eudragit suspension. The Wurster insert is used to apply the coating suspension.

### Second Overcoat

Once the retardant coating is complete, the hydromorphone beads are given a final overcoat of Opadry Light Pink to a 5% weight gain using a Wurster insert. This overcoat is also applied as a protective coating.

### Curing

After the completion of the final overcoat, the hydromorphone beads are cured in a 45° C. oven for 2 days.

### Encapsulation

The beads are mixed with shogaol in a suitable mixer and the mixture is then hand filled in size #2 clear gelatin capsules.

### EXAMPLE 4 30 mg extended release oxycodone tablets using piperine

Tablets are prepared as described in Example 1, except that the respiratory irritiant used is piperine, and the quantities used are as follows:

| Ingredient | mg/tabl et |
|---|---|
| Oxycodone HCl | 30 |
| Lactose | 138.75 |
| Eudragit® RS PM | 45 |
| Purified water | as needed |
| Stearyl alcohol | 75 |
| Talc | 7.5 |
| Magnesium stearate | 3.75 |
| Piperine | 75 |

### EXAMPLE 5 30mg Extended release morphine sulfate tablets using denatonium benzoate

These tablets are prepared as described in Example 2 except that the capsaicin is replaced with 2mg/tablet of denatonium bezoate (available as Bitrex® from Macfarlan Smith, Edinburgh, UK).

### EXAMPLE 6

A Caucasian female, age 27 , weighing 50 kg suffering from back pain takes a 30mg tablet of sustained release oxycodone from Example 1 as prescribed, by swallowing said tablet whole. She experiences 15 hours of pain relief without any aversive response.

### EXAMPLE 7

A Caucasian male recreational drug user, age 45, weighing 80 kg crushes a 30mg tablet of sustained release oxycodone from Example 1 to give a powder. He then inhales said powder through the nose. He immediately experiences an aversive response, including an intense burning sensation in the nasal passages, sneezing, watery eyes and headache.

### EXAMPLE 8

An African-American male, aged 18, weighing 66 kg, who has never abused drugs crushes an extended release 30mg morphine tablet from Example 2 into a powder and then inhales said powder through the nose. He immediately experiences an aversive response, including an intense burning sensation in the nasal passages, coughing, and watery eyes.

### EXAMPLE 9

An Asian female, recreational drug user, aged 28, weighing 55 kg, breaks an 8mg hydromorhone capsule from Example 3 and empties out the contents from the capsule. She crushes the beads into a fine powder and then inhales said powder through the nose. She immediately experiences an aversive response, including an intense burning sensation in the nasal passages and the throat, coughing, and watery eyes.

### EXAMPLE 10

At a party a Caucasian male recreational drug user, aged 17, weighing 65 kg is offered a 30mg morphine sulfate tablet, as prepared in Example 5 . He chews it and he immediately experiences an aversive response causing him to spit out the chewed dosage form.

## Claims

1. An oral pharmaceutical dosage form not susceptible to abuse by respiratory mucosal membrane administration comprising one or more aversive agents.

2. An oral pharmaceutical dosage form not susceptible to abuse by chewing comprising one or more aversive agents.

3. An oral pharmaceutical dosage form not susceptible to abuse by respiratory mucosal membrane administration comprising one or more aversive agents and a controlled substance.

4. An oral pharmaceutical dosage form not susceptible to abuse by chewing comprising one or more aversive agents and a controlled substance.

5. An oral pharmaceutical dosage form according to Claim 3, wherein said aversive agent is a respiratory irritant selected from the group consisting of capsaicin, capsacutin dihydrocapsaicin, nordihydrocapsaicin, homocapsaicin, homodihydrocapsaicin, capsaicinoids, gingerol, pipeline, isopiperine, zingerone, shogaol, and vanyllylamide derivatives, and mixtures thereof.

6. An oral pharmaceutical dosage form according to Claim 4, wherein said aversive agent is a bitter substance selected from the group consisting of denatonium benzoate, denatonium capsaicinate, denatonium chloride, denatonium saccharide, limonin, linalool, linalool acetate, naringin, quassin, quercitin, sucrose benzoate, and sucrose octaacetate, and mixtures thereof.

7. An oral pharmaceutical dosage form according to Claim 5, wherein said controlled substance is selected from the group consisting of Schedule II, III, IV, and V controlled substances.

8. An oral pharmaceutical dosage form according to Claim 6, wherein said controlled substance is selected from the group consisting of Schedule II, III, IV, and V controlled substances.

9. An oral pharmaceutical dosage form according to Claim 7, wherein said controlled substance is a Schedule II controlled substance.

10. An oral pharmaceutical dosage form according to Claim 8, wherein said controlled substance is a Schedule II controlled substance.
